# EUROPEAN PATENT APPLICATION

(11) **EP 2 172 538 A2**
(43) Date of publication of application: **07.04.2010**
(21) Application number: 09161564.1
(22) Date of filing: 29.05.2009
(51) Int. Cl.: C12M 1/22

(54) **Holding petri dish, microinjection apparatus, and microinjection method**

(30) Priority: 13.08.2008 JP 2008208347
(71) Applicant: Fujitsu Limited, Kawasaki-shi, Kanagawa 211-8588 (JP)
(72) Inventor: Nishiyama, Shusaku, Nakahara-ku, Kawasaki-shi, Kanagawa 211-8588 (JP); Nishio, Chikara, Nakahara-ku, Kawasaki-shi, Kanagawa 211-8588 (JP)
(74) Representative: Stebbing, Timothy Charles

(57) **Abstract**

A holding petri dish (1) used for a process of microinjection for introducing an introduced substance into an introduced-substance receiving body (3) includes a well (2) provided on a transparent material (7) and a channel (5) including an opening (5a) for holding the introduced-substance receiving body (3) via suction, said opening (5a) being disposed on a side wall (2a) of the well (2).

## Description

The embodiments discussed herein are related to a holding petri dish used for a process of microinjection in which a liquid substance or the like (introduced substance) obtained by mixing a medicine, etc., into an intermediary and by diffusing the medicine, etc., in the intermediary is introduced into a minute (small) living body or the like such as a cell, etc. (an introduced-substance receiving body), and to a microinjection apparatus and a microinjection method.

In the conventional research fields of medical science, genetic engineering, and drug design, etc., in order to introduce a substance into a cell, a process has been performed in which a suction nozzle is used for holding, via suction, an introduced-substance receiving body such as a cell or the like in a holding petri dish or the like, a micro needle filled with an introduced substance such as a medicine is inserted from the side opposed to the suctioning side, and the introduced substance is discharged in order to introduce the substance into the introduced-substance receiving body. This process has been performed manually by a person using a microscope to observe the entire process (See Patent Documents 1 through 3 for example).

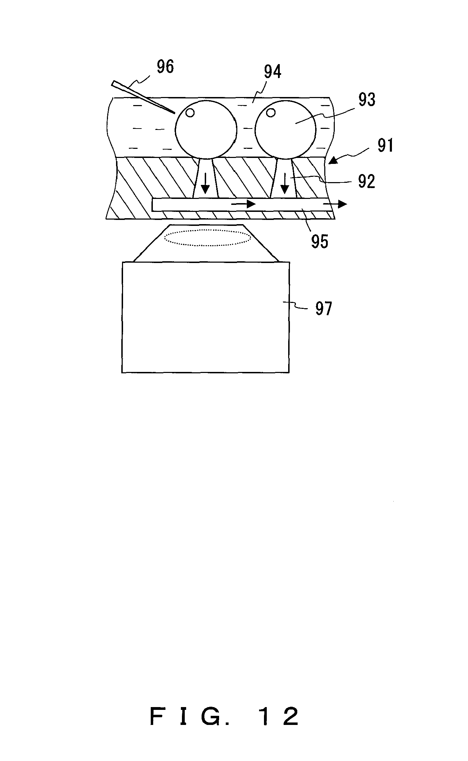

Fig. 12 is a cross-sectional view depicting the main part of a conventional holding petri dish.

A holding petri dish 91 depicted in Fig. 12 has a plurality of micro suction holes 92 formed on the top surface. The suction holes 92 are in communication with a channel 95 formed in the holding petri dish 91. A mouse-egg cell 93 is held via suction in the suction holes 92 with a culture fluid 94 being suctioned through the channel 95.

The image of the mouse-egg cell 93 being held via suction is enlarged and observed with a microscope 97, and an introduced substance such as genes, etc., is introduced into the mouse-egg cell 93 through a capillary 96.

Generally, the suction holes 92 are formed on a silicon substrate (the holding petri dish 91) using techniques of MEMS (Micro Electro Mechanical Systems) such as photolithography or etching.

However, the use of a silicon substrate as a holding petri dish prevents the transmission observation, which uses visible light, of the cells in micro suction holes. Also, the transmission imaging using infrared rays requires that a silicon substrate be at most 10µm in thickness.

Further, in the introduction of a gene into a cell, the introduction site (such as a pronucleus) can be recognized effectively by picking up an image of it using a differential interference optical system while images obtained by this method are blurred when the path of the visible light (below the holding position in the example in Fig. 12) involves a structure having an irregular shape due to the existence of micro suction holes for holding cells.
Patent Document 1:
   Japanese Laid-open Patent Publication No. 2006-280231
Patent Document 2:
   Japanese Laid-open Patent Publication No. 2006-109715
Patent Document 3:
   Japanese Laid-open Patent Publication No. 2007-222132

In view of the above conventional situations, it is desirable to provide a holding petri dish, a microinjection apparatus, and a microinjection method that permit the picking up of a clear image of an introduced-substance receiving body held via suction.

According to an aspect of the invention, there is provided a holding petri dish used for a process of microinjection for introducing an introduced substance into an introduced-substance receiving body, comprising: a well provided on a transparent material; and a channel including an opening for holding the introduced-substance receiving body via suction, the opening being disposed on a side wall of the well.

According to another aspect of the invention, there is provided a microinjection apparatus for introducing an introduced substance into an introduced-substance receiving body, comprising: a holding petri dish including a well provided on a transparent material, and a channel having an opening for holding the introduced-substance receiving body, the opening being disposed on a side wall of the well; a suction mechanism for holding the introduced-substance receiving body on the opening of the well via suction through the channel; and a transmission observation optical system for performing transmission observation of the introduced-substance receiving body.

According to a further aspect of the invention, there is provided a microinjection method of introducing an introduced substance into an introduced-substance receiving body, comprising: a suctioning step of holding, via suction, the introduced-substance receiving body on an opening provided on a side wall of a well, the introduced-substance receiving body having been guided to the well on a transparent material in a holding petri dish; and an observing step of performing transmission observation of the introduced-substance receiving body held on the opening of the well.

The advantages of the invention will be realized and attained by means of the elements and combinations particularly pointed out in the claims.

It is to be understood that both the foregoing general description and the following detailed description are exemplary and explanatory and are not restrictive of the invention, as claimed.

Embodiments of the present invention will now be described with reference to the accompanying drawings, of which:
Fig. 1 is a cross-sectional view depicting the main part of a holding petri dish according to the first embodiment of the present invention;
Fig. 2A depicts an image of an egg cell on a holding petri dish according to the first embodiment of the present invention;
Fig. 2B depicts an image of an egg cell on a conventional holding petri dish;
Fig. 3 is a cross-sectional view depicting the main part of a holding petri dish according to the second embodiment of the present invention;
Fig. 4A is a perspective view depicting a well of the holding petri dish according to the second embodiment;
Fig. 4B is a perspective view depicting a well of a holding petri dish according to the first variation of the second embodiment;
Fig. 4C is a perspective view depicting a well of a holding petri dish according to the second variation of the second embodiment;
Fig. 4D is a perspective view depicting a well of a holding petri dish according to the third variation of the second embodiment;
Fig. 5 is a cross-sectional view depicting a holding petri dish according the third embodiment;
Fig. 6A is an enlarged view partially depicting a microinjection apparatus according to the fourth embodiment of the present invention;
Fig. 6B is a perspective view schematically depicting the microinjection apparatus according to the fourth embodiment of the present invention;
Fig. 7 is a cross-sectional view explaining the adjustment of the orientation of an egg cell by the microinjection apparatus according to the fourth embodiment of the present invention;
Fig. 8 is an enlarged view partially depicting a microinjection apparatus according to the fifth embodiment;
Fig. 9 is a cross-sectional view depicting the main part of a microinjection apparatus according to the sixth embodiment;
Fig. 10 is a cross-sectional view depicting the main part of a microinjection apparatus according to the seventh embodiment;
Fig. 11 depicts the entire configuration of a microinjection apparatus according to the eighth embodiment of the present invention; and
Fig. 12 is a cross-sectional view depicting the main part of a conventional holding petri dish.

Hereinafter, a holding petri dish, a microinjection apparatus, and a microinjection method according to embodiments of the present invention will be explained by referring to drawings.

### <First Embodiment>

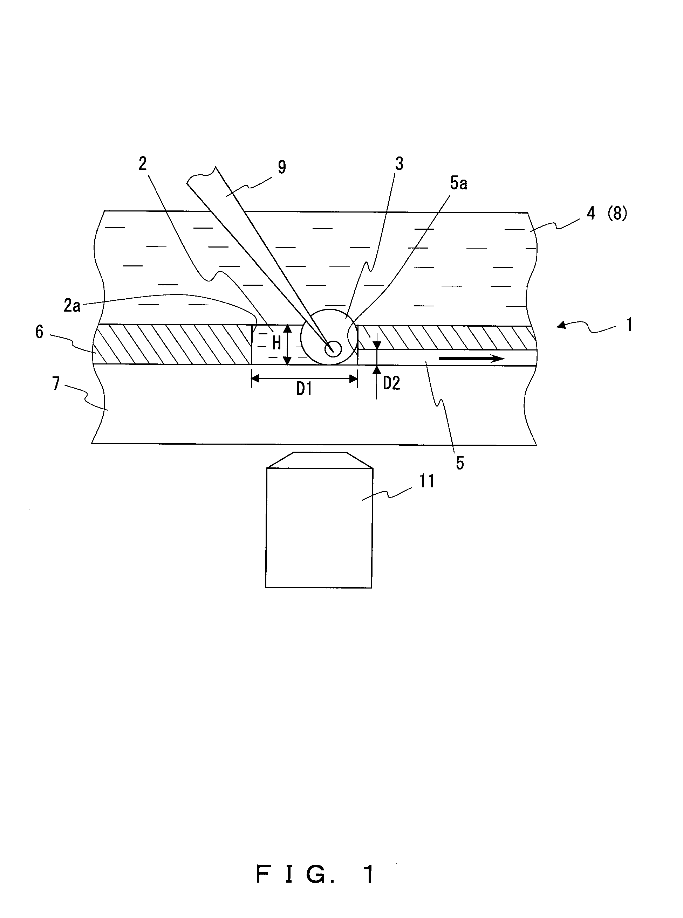

Fig. 1 is a cross-sectional view depicting the main part of a holding petri dish 1 according to the first embodiment of the present invention.

The holding petri dish 1 depicted in Fig. 1 is made of a transparent material such as plastic (resin), glass or the like, and has wells 2 formed on the top surface. Upon the introduction into a knockout mouse, genes (introduced substances) are introduced into ten through several tens of egg cells (introduced-substance receiving bodies) 3 typically in a single experiment. For this reason, for example, ten through several tens of wells 2 are formed in a culture fluid bath 8 to which a culture fluid (liquid) 4 is supplied as an intermediary.

It is desirable that the holding petri dish 1 be configured to have at least a portion below the wells 2 be made of a transparent material and to involve no channels or no irregularities in shape such as unevenness so as to permit transmission observation from below the wells 2 using a microscope (objective lens) 11. In the example in Fig. 1, the holding petri dish 1 is made entirely of a transparent material.

Each of the wells 2 is in the shape of a cylinder having a diameter D1 that is 1.5 to 5 times greater than the diameter (100µm through 200µm) of the egg cell 3 and having a height H equal to or slightly greater than the radius of the egg cell 3.

An opening 5a of the channel 5 is formed on a side wall 2a of each of the wells 2. This opening 5a is formed so that a suction mechanism (not depicted) holds, via suction through the channel 5, the egg cell 3 in the well 2. The channel 5 in the present embodiment has a circular vertical cross section.

When stability in the holding of the egg cell 3 is a priority, the opening 5a of the channel 5 is desirably provided at a level corresponding to the level of the center of the egg cell 3. However, when an introduction site (pronucleus) 3a is much smaller than the outer surface of the cell as in the case of the egg cell 3 of a mouse, and the adjustment of the orientation is a priority, the opening 5a is desirably provided at the level of the bottom of the well 2 so that the suction (or the discharging to be described later) can easily rotate the egg cell 3. It is desirable that the opening 5a have a diameter D2 (or height/width) that is about 1/4 through 1/2 the size of the introduced-substance receiving body (the diameter of the egg cell 3).

The base part, which is a part of the holding petri dish 1 and on which the wells 2 and the channel 5 are formed, includes an upper plate 6 and a lower plate 7 formed under the upper plate 6. The upper plate 6 has the wells 2 piercing it and the channel 5 formed on the bottom of it.

The upper plate 6 and the lower plate 7 are appropriately jointed together using the technique of diffusion junction when the channel 5 is thin, although they may be jointed together using other methods including adhesion, ultrasonic junction, diffusion junction, etc., when they are made of plastic. Also, when the upper plate 6 and the lower plate 7 are made of glass, adhesion, welding junction, anodic bonding, etc., may be used, and anodic bonding is the most suitable.

When the upper plate 6 and the lower plate 7 is made of PDMS (polydimethysiloxane) and the other of them is made of glass, the junction at normal temperature (Si-O-Si) conducted after performing surface activation is suitable.

The configurations and the operations of the microinjection apparatus will be explained in embodiments that are to be described later, and hereinafter the points in the introduction of genes into the egg cells 3 that relate to the wells 2 and the channel 5 in the holding petri dish 1 are explained.

First, the culture fluid 4 is supplied to the culture fluid bath 8, and the well 2 becomes full of the culture fluid 4. Next, the egg cell 3 is set in the well 2. Upon this setting, the suction mechanism (not depicted) in this microinjection apparatus holds the egg cell 3 on the opening 5a via suction through the channel 5. When the diameter L2 of the channel 5 is, for example, approximately 30 µm, the flow rate of the fluid suctioned by the suction mechanism is desirably on the order of several nl per minute.

Then, a gene is introduced into the introduction site 3a with a capillary 12 while the transmission observation of the egg cell 3 held on the opening 5a is being performed using the microscope 11. This introduction of genes is repeated with the microscope 11, the capillary 12, etc. being moved sequentially to the respective wells 2.

In the present embodiment described above, the egg cell 3 is held via suction on the opening 5a formed on the side wall 2a of each of the wells 2 on a transparent material. This makes it possible to avoid a situation in which the channel 5 or an opaque material prevents the observation of the egg cell 3 when picking up an image of the egg cell 3 held on the opening 5a. Accordingly, the present embodiment enables the picking up of a clear image of the egg cell 3 held via suction.

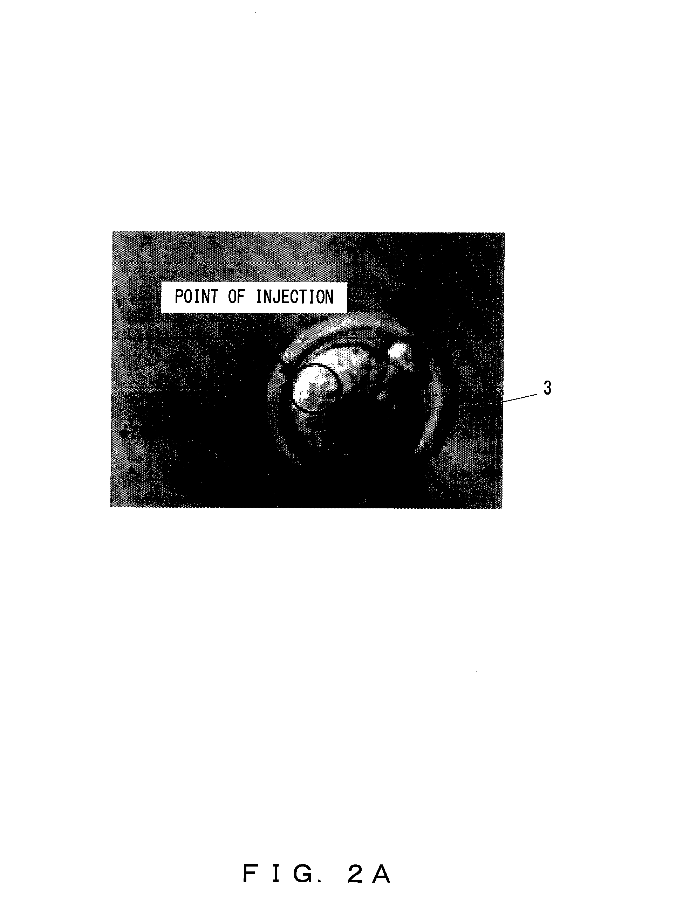

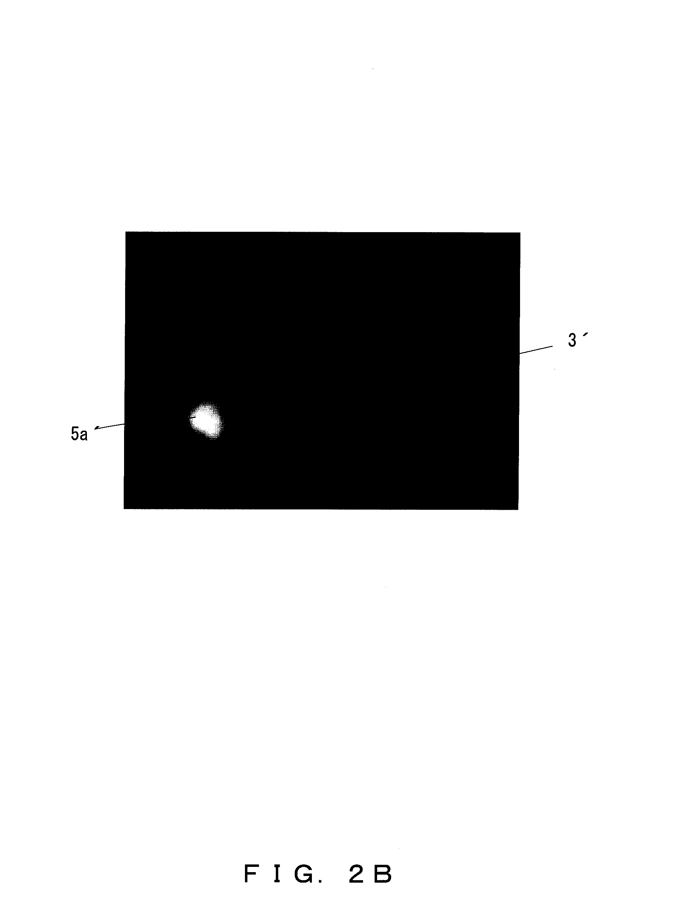

As depicted in Fig. 2A, the present embodiment enables the picking up of a clearer image of the egg cell 3 than the observation image of an egg cell 3' depicted in Fig. 2B, which is obtained by using a conventional petri dish having a suction hole 5a' formed on the bottom surface of a well made of silicon.

It is to be noted that although an example in which each of the wells 2 is cylindrical has been explained in the present embodiment, the wells 2 can be in any shape as long as they function as concavities. Further, it is desirable that the opening 5a of the channel 5 be formed on the side wall 2a of each of the wells 2, e.g., a curved or flat plane crossing the horizontal plane.

In addition, although an example is given in which egg cells 3 are used as introduced-substance receiving bodies, introduced-substance receiving bodies other than the egg cells 3 including a minute living body such as a cell may also be used.

### <Second Embodiment>

The present embodiment is different from the first embodiment only in the shape of wells 22, and is approximately the same as the first embodiment in other points. Thus, the same or similar parts (elements) as in the first embodiment will be denoted by the same symbols as in the first embodiment, and the explanations thereof will be omitted.

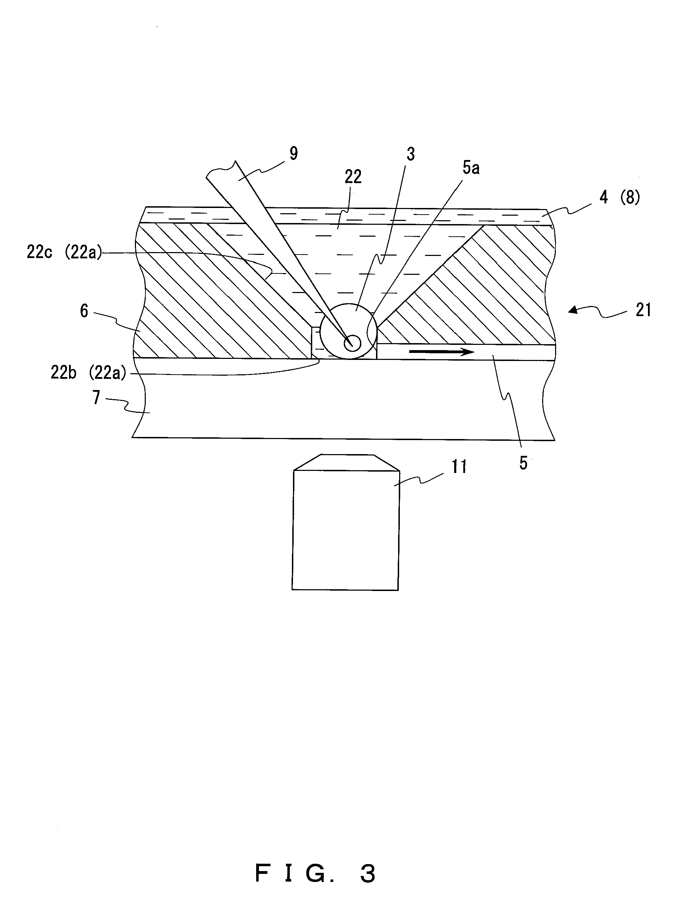

Fig. 3 is a cross-sectional view depicting the main part of a holding petri dish 21 according to the second embodiment of the present invention.

In a well 22 in the holding petri dish 21 depicted in Fig. 3, a lower part 22b of the side wall 22a is cylindrical (i.e., has a vertical wall), and an upper part 22c is in a shape of a cone (i.e., has an inclined plane wall) with its diameter increasing gradually with increasing distance from the bottom to the top. The surface of the inclined plane of the well 22 is smooth so that the egg cell 3 cannot get stuck, and needs to be at an angle to allow the egg cell 3 to roll down. For this purpose, the inclined plane of the upper part 22c is desirably at least 30 degrees with respect to the horizontal plane so as to ensure the rolling of the egg cell 3.

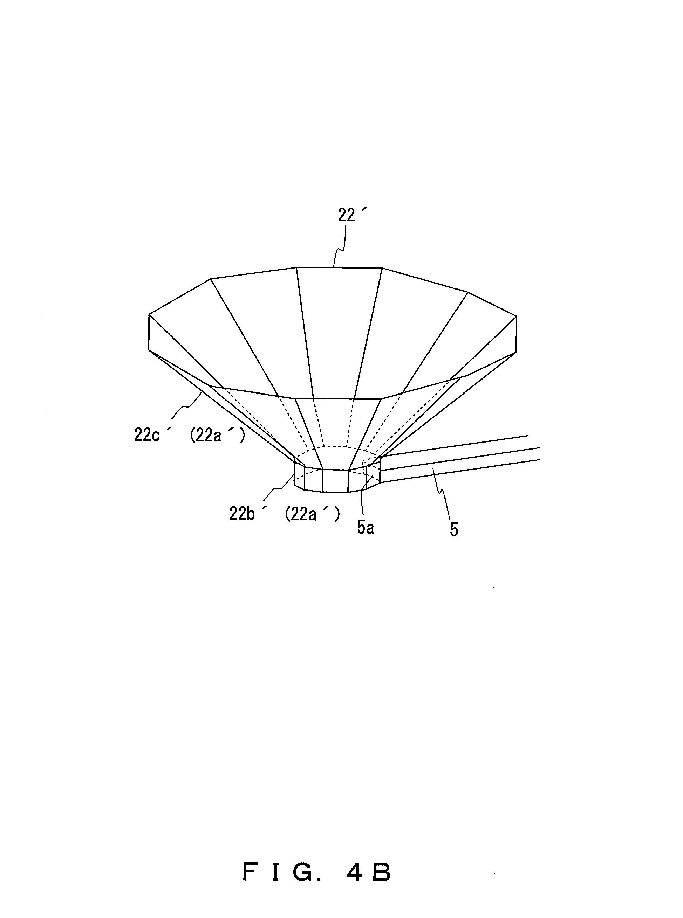

Further, other planes including, for example, the inclined planes of the multi-sided pyramid (twelve sided pyramid) depicted in Fig. 4B, the free-form surface, or the inclined plane of a hemisphere can be used as the inclined planes for the upper part 22c.

When the inclined planes of the multi-sided pyramid (twelve sided pyramid) depicted in Fig. 4B are used for an upper part 22c', a lower part 22b' is desirably a polygonal column having as many corners as the multi-sided pyramid.

Further, it is also possible to employ, as one part of an upper part 22c", an inclined plane 22c''-1 whose diameter gradually increases with increasing distance from the bottom toward the top, and to dispose another part of the upper part 22c'' on the side of the opening 5a of the channel 5 while forming that part into a vertical plane 22c"-2 opposite to the inclined plane 22c''-1 so that the inclined plane 22c''-1 and the vertical plane 22c"-2 form a semi-elliptical cone. Also, instead of the vertical plane 22c''-2, an inclined plane with an angle smaller than that of the inclined plane 22c''-1 with respect to the vertical plane 22c"-2 may be employed. In such a case, the inclined plane may be inclined in either the same direction or in the other direction with respect to the inclined plane 22c''-1, depending upon the shape of the well 22.

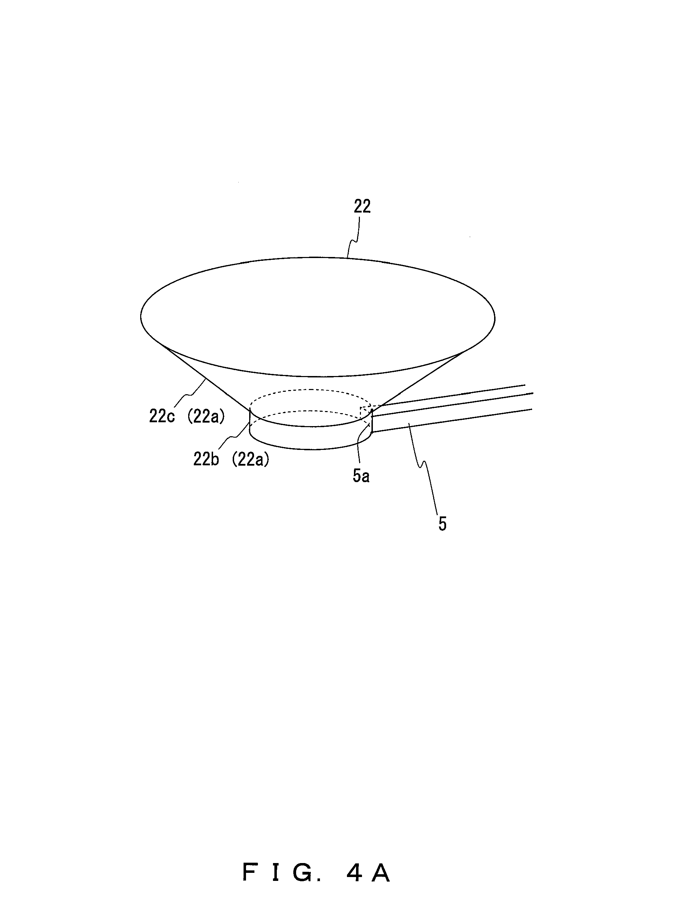

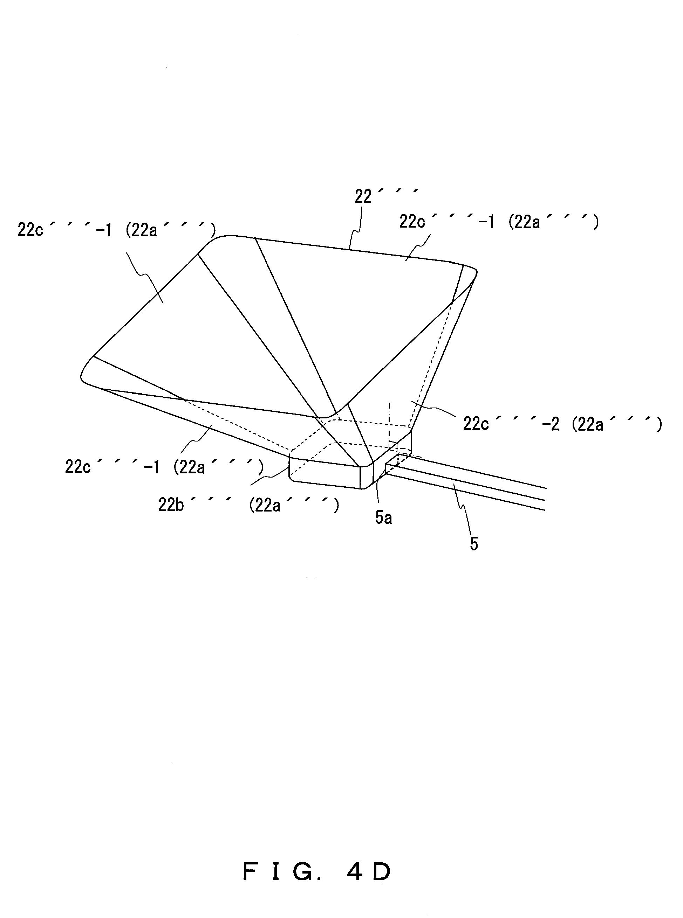

It is also possible to form, as depicted in Fig. 4D, a semi-multi-sided pyramid having three inclined planes 22c'''-1 that extend outwardly with increasing distance from the bottom toward the top, and one vertical plane 22c'''-2.

Also in the present embodiment explained above, the egg cell 3 is held via suction on the opening 5a formed on the side wall 22a of the well 22 provided on a transparent material. Accordingly, the present embodiment as well enables the picking up of a clear image of the egg cell 3 held via suction.

In addition, in the present embodiment, the side wall 22a of the well 22 has an inclined plane (the upper part 22c) whose diameter gradually increases with increasing distance from the bottom toward the top, and a vertical plane (the lower part 22b) disposed below the inclined plane and having an opening 5a. This makes it possible to guide the egg cell 3 to the opening 5a by, for example, rolling it.

Also, in the present embodiment a side wall 22a' of the well 22' is at least partially made of a multi-sided pyramid whose diameter gradually increases with increasing distance from the bottom toward the top (as depicted in Fig. 4B) or is made of a curved and inclined plane. This also makes it possible to guide the egg cell 3 to the opening 5a by, for example, rolling it.

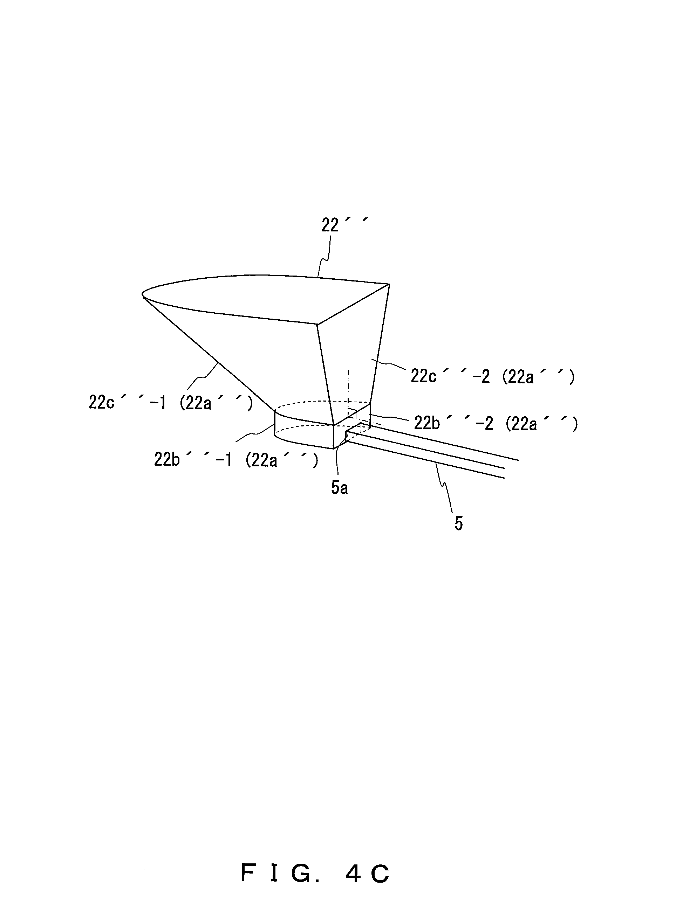

Also, in the present embodiment side walls 22a" and 22a''' include, as depicted in Figs. 4C and 4D, the inclined planes 22c''-1 and 22c'''-1 each having a diameter gradually increasing with increasing distance from the bottom toward the top, and also include the planes 22c''-2 and 22c'''-2 being disposed on the side of the opening 5a of the channel 5 and being opposite to the above inclined planes 22c''-1 and 22c'''-1. Further, these planes are planes with angles smaller than the angles of the above inclined planes 22c''-1 and 22c'''-1 with respect to the vertical planes 22c"-2 and 22c"'-2 or the vertical planes 22c''-2 and 22c"'-2.

The above configuration can effectively guide the egg cell 3 to the opening 5a by, for example, rolling it while using the wells 22" and 22''' that are not large.

Also, in the present embodiment, the inclined planes 22c''-1 and 22c'''-1 and the vertical planes 22c''-2 and 22c'''-2 form a semi-elliptical cone (as depicted in Fig. 4C) or a semi-multi-sided pyramid with corners R (as depicted in Fig. 4D). This configuration can effectively guide the egg cell 3 to the opening 5a by, for example, rolling it.

### <Third Embodiment>

In the explanation of the present embodiment, the same or similar parts (elements) as in the first or second embodiments are denoted by the same symbols as in those embodiments, and the explanations thereof will be omitted.

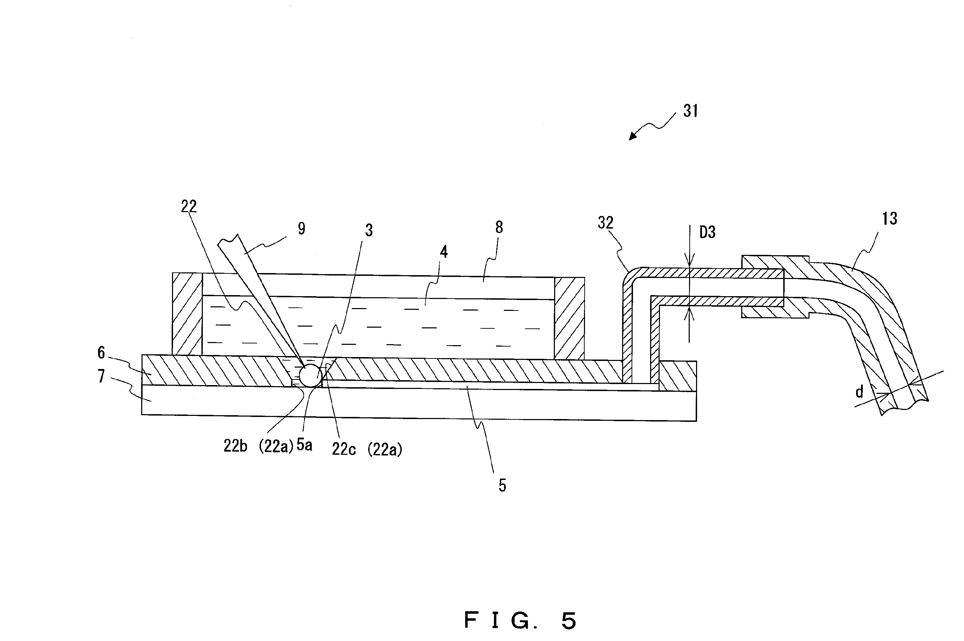

Fig. 5 is a cross-sectional view depicting a holding petri dish 31 according the third embodiment.

The holding petri dish 31 depicted in Fig. 5 includes a connection unit 32 disposed between silicon tube 13 (an elastic pipe on the side of a suction mechanism side) and the channel 5 of the holding petri dish 31. The silicon tube 13 is connected to a suction mechanism (not depicted) that holds the egg cell 3 via suction through the channel 5. The connection unit 32 is a tube made of a hard material such as stainless steel or the like, and is, in the present embodiment, bent at 90 degrees at the middle in the longitudinal direction.

The connection unit 32 is fit into, for example, the upper plate 6 of the holding petri dish 31 from above, and is in communication with the channel 5. Also, the connection unit 32 is press-fitted into the silicon tube 13.

The outer diameter D3 of the connection unit 32 is approximately 1.5 times greater than the inner diameter of the silicon tube 13.

Also in the present example as well, the egg cell 3 is held via suction on the opening 5a formed on the side wall 22a of the well 22 provided on a transparent material. Thus, the present embodiment as well enables the picking up of a clear image of the egg cell 3 being held via suction.

Also, in the present embodiment, the holding petri dish 31 includes the connection unit 32 between the silicon tube (elastic pipe on the side of the suction mechanism) 13 and the channel 5. Further, the connection unit 32 is a pipe made of a hard material.

This ensures the connection between the silicon tube 13 and the connection unit 32, preventing the silicon tube 13 from being disconnected from the holding petri dish 31.

Also, in the present embodiment, the outer diameter D1 of the connection unit 32 is approximately 1.5 times greater than the inner diameter d of the silicon tube 13. This effectively prevents the silicon tube 13 from being disconnected from the holding petri dish 31 while suctioning, etc.

### <Fourth Embodiment>

In the explanation of the present embodiment, the same or similar parts (elements) as in the first through third embodiments are denoted by the same symbols as in those embodiments, and the explanations thereof will be omitted.

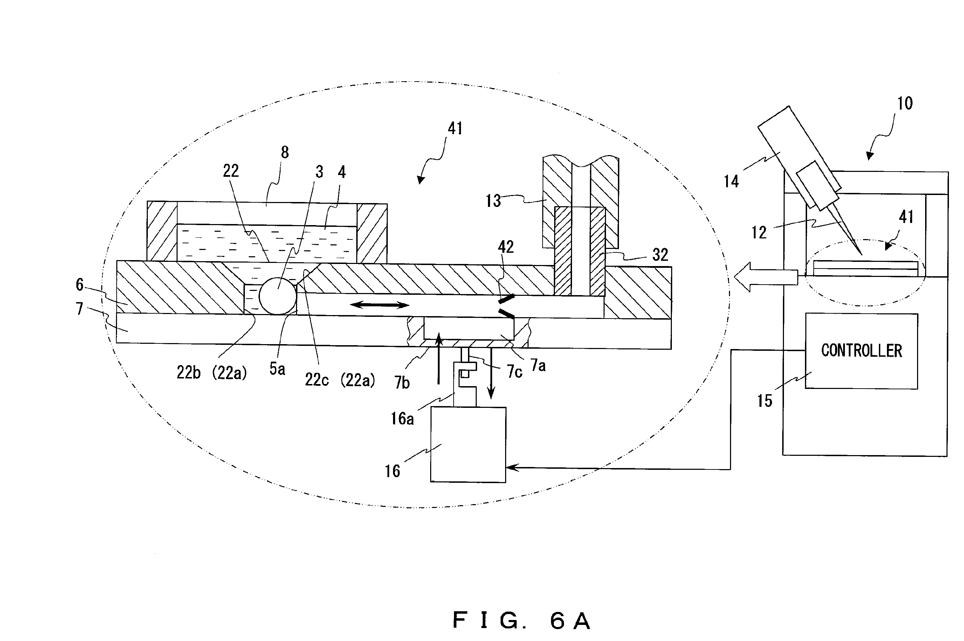

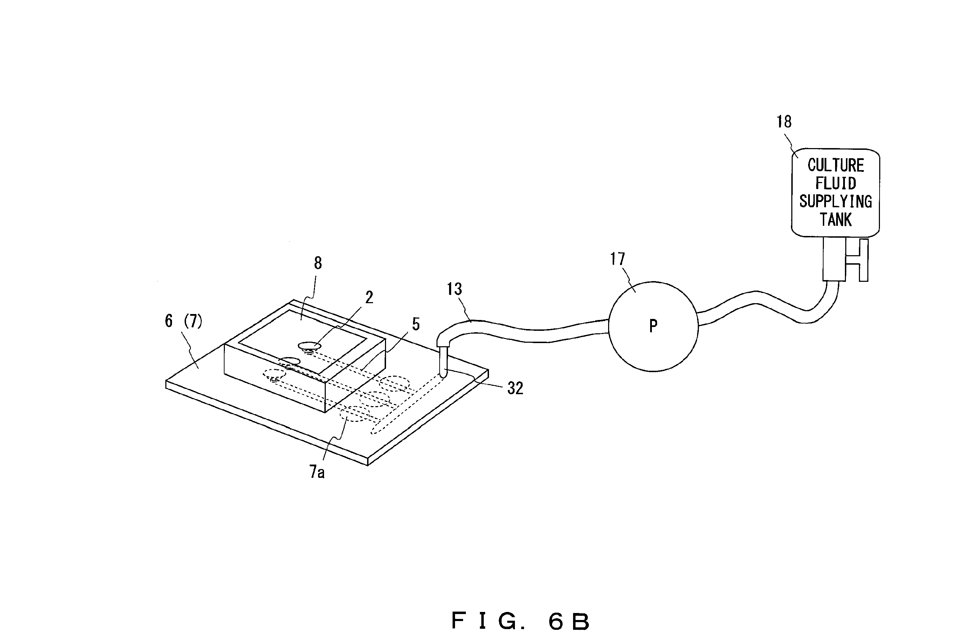

Fig. 6A is an enlarged view partially depicting a microinjection apparatus 10 according to the fourth embodiment of the present invention. Fig. 6B is a perspective view schematically depicting the microinjection apparatus 10.

The microinjection apparatus 10 includes a holding petri dish 41, the capillary 12, a manipulator 14 with the capillary 12 at its tip, a controller 15, an actuator 16 functioning as both the suction mechanism and the discharge mechanism, a suction/discharge pump 17, a culture fluid supplying tank 18, and the like.

On the lower plate 7 of the holding petri dish 41, there is a concave part 7a creating space integrated with the channel 5 provided on the bottom surface of the upper plate 6. Below the bottom of the concave part 7a, there is a membrane part 7b having its thickness made smaller by the provision of the concave part 7a. Below the membrane part 7b, there is a pin 7c projecting from the membrane part 7b in exactly the downward direction to be integrated with the membrane part 7b.

The actuator 16 is provided with an elevating unit 16a that moves up and down while holding the pin 7c. The actuator 16 moves the pin 7c up and down by moving the elevating unit 16a up and down through control by the controller 15. The upward and downward movement of the pin 7c pushes up and pulls down the membrane part 7b.

The upward and downward movement of the membrane part 7b changes the volume of the concave part 7a. When the membrane part 7b is pulled down to increase the volume of the concave part 7a, the culture fluid 4 flows into the channel 5 from the well 22 so as to hold the egg cell 3 via suction on the opening 5a. When the membrane part 7b is pushed up to decrease the volume of the concave part 7a, the culture fluid 4 flows into the well 22 from the channel 5 to be discharged toward the egg cell 3.

The concave part 7a, the membrane part 7b, and the pin 7c are provided to each channel 5. In addition, a check valve 42 is provided to each channel 5 so as to prevent the culture fluid 4 from being caused by the upward and downward movement of the membrane part 7b to flow into the side of the suction/discharge pump 17.

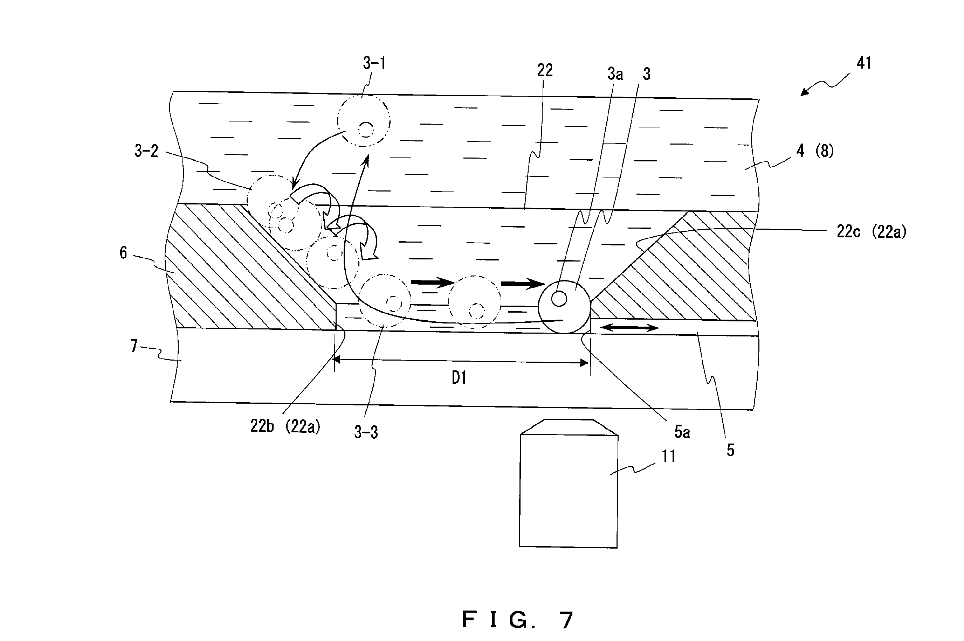

The diameter (width) D1 of the part having the opening 5a in the well 22 is, as depicted in Fig. 7, 1.5 through 5 times greater than the diameter (size) of the egg cell 3 (introduced-substance receiving body).

When the transmission observation by the microscope 11 determines the orientation (the position of the introduction site 3a) of the egg cell 3 being held via suction to be unfavorable to the introduction of genes to the egg cell 3, the check valve 42 closes the channel 5 first.

Then, the actuator 16 moves the pin 7c (the membrane part 7b) upward in order to make the culture fluid 4 in the channel 5 flow into the well 22 so as to release the egg cell 3 from the opening 5a. Thereby, the egg cell 3 floats in the culture fluid bath 8 (egg cell 3-1). Additionally, it is desirable that the culture fluid 4 be discharged at the rate of several nl in a pulse manner when the introduced-substance receiving body is the egg cell 3 of a mouse.

After being released from the opening 5a, which is the holding part, and floating in the culture fluid bath 8, the egg cell 3 sinks and rolls down along an inclined plane (the lower part 22b) provided on the side wall 22a of the well 22 (egg cell 3-2).

Thereafter, the actuator 16 moves the pin 7c (the membrane part 7b) downward so as to suction into the channel 5 the culture fluid 4 in the well 22. Thereby, the egg cell 3 moves along the bottom surface of the well 22 (egg cell 3-3), and is again held on the opening 5a via suction.

When the egg cell 3 is in a desirable orientation after the above release and the repeated holding of the egg cell 3, a gene is introduced into the egg cell 3 using the capillary 12 of the manipulator 14. When the egg cell 3 is not in a desirable orientation, the release and the repeated holding are performed again.

Also in the above described embodiment as well, the egg cell 3 is held via suction on the opening 5a formed on the side wall 22a of the well 22 provided on a transparent material. Thus, the present embodiment as well enables the picking up of a clear image of the egg cell 3 being held via suction.

Also, in the present embodiment the actuator 16 holds the egg cell 3 via suction through the channel 5 on the opening 5a of the well 22 and discharges the culture fluid 4 toward the egg cell 3.

This makes it possible to release the suctioned egg cell 3 from the opening 5a, which is the holding part, and to again hold the egg cell 3 via suction so that the egg cell 3 can be held in a different orientation. There has been a general problem wherein when the egg cell 3 being held does not have its introduction site 3a into which the injection is to be performed facing the side of the capillary 12, the contents in the cell are pushed by a deformation or the like caused by the insertion of the capillary 12 so that the introduction site 3a is moved, the introduction position is shifted, or the introduction site 3a is damaged. However, the present embodiment can eliminate this problem of damaging the introduction site 3a by changing the orientation of the egg cell 3 when being held.

Also, the well 22 of the holding petri dish 41 in the present embodiment has the part with the opening 5a, and the diameter of that part is 1.5 through 5 times greater than the diameter of the egg cell 3. This ensures the suctioning by the suction/discharge pump 17 of the egg cell 3 that was released once. Thereby, the egg cell 3 can effectively be held in a different orientation.

Also, in the present embodiment the liquid is suctioned and discharged in the same channel, i.e., the channel 5. This makes it possible to provide to the holding petri dish 41 greater numbers of the wells 2 and the channels 5.

Also, in the present embodiment the actuator 16 for suctioning and discharging the liquid is provided to each of a plurality of channels 5. This makes it possible to control the suctioning and discharging independently in units of the channels 5.

Also, the present invention includes the check valve 42 in each of the channels 5 in the holding petri dish 41. This enables the actuator 16 to effectively suction and discharge the liquid.

### <Fifth Embodiment>

In the explanation of the present embodiment, the same or similar parts (elements) as in the first through fourth embodiments are denoted by the same symbols as in those embodiments, and the explanations thereof will be omitted.

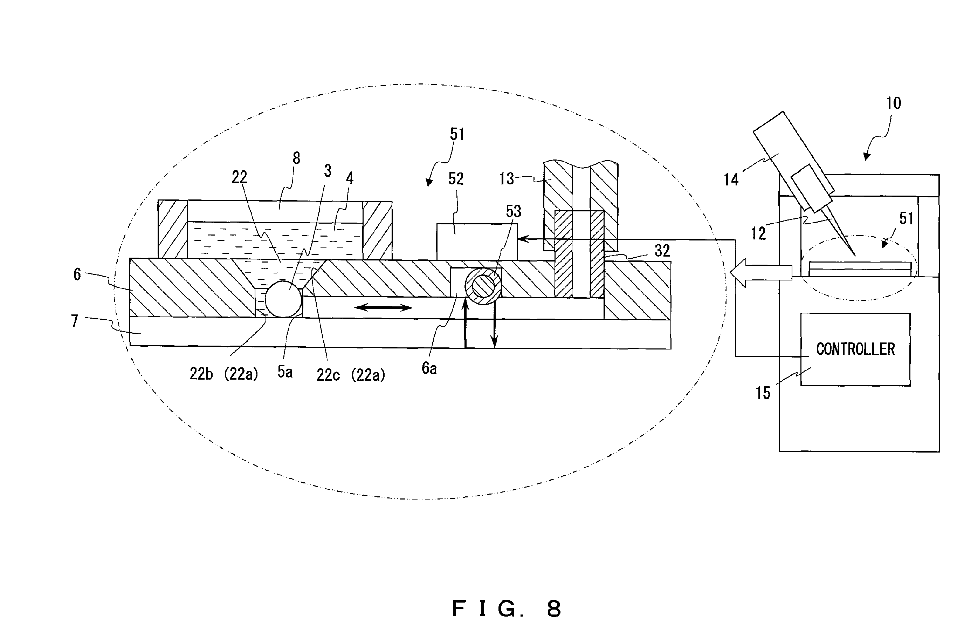

Fig. 8 is an enlarged view partially depicting the microinjection apparatus 10 according to the fifth embodiment of the present invention.

In the present embodiment, a concave part 6a creating space integrated with the channel 5 is provided on the bottom surface of the upper plate 6 of a holding petri dish 51 of the microinjection apparatus 10 according to the present embodiment. An electromagnet 52 is provided above the concave part 6a and on the upper plate 6. A valve 53 that is moved between the concave part 6a and the channel 5 by the magnetic force of the electromagnet 52 is disposed in the concave part 6a. The electromagnet 52 and the valve 53 are provided to each of the channels 5 and function as a solenoid valve, which is an opening/closing mechanism.

The valve 53 is constituted of a magnet and a plastic material coated on the magnet. The valve 53 is accommodated in the concave part 6a with an electric pressure applied to itself under control by the controller 15, and moves between the position at which it does not prevent the flow of the culture fluid 4 in the channel 5 and the position at which it closes the channel 5 to prevent the flow of the culture fluid 4 in it.

Also in the present embodiment, the egg cell 3 is held via suction on the opening 5a formed on the side wall 22a of the well 22 provided on a transparent material. Accordingly, the present embodiment as well enables the picking up of a clear image of the egg cell 3 held via suction.

Also, the valve 53 is moved by the magnetic force of the electromagnet 52 between the position at which it does not prevent the flow of the culture fluid 4 in the channel 5 and the position at which it closes the channel 5 to prevent the flow of the culture fluid 4 in it. Thereby, the control of the suctioning and discharging can be performed only in a desired channel of the channels 5 with less suction/discharge mechanisms than the channels 5.

### <Sixth Embodiment>

In the explanation of the present embodiment, the same or similar parts (elements) as in the first through fifth embodiments are denoted by the same symbols as in those embodiments, and the explanations thereof will be omitted.

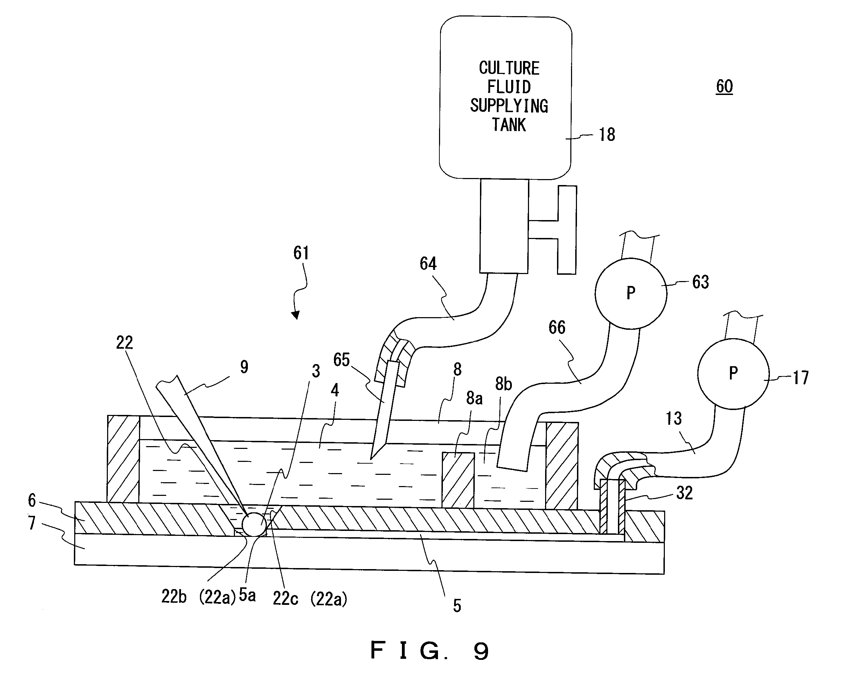

Fig. 9 is a cross-sectional view depicting the main part of a microinjection apparatus 60 according to the sixth embodiment.

The microinjection apparatus 60 according to the present embodiment includes a holding petri dish 61, the silicon tube 13, the suction/discharge pump 17, a culture fluid supply tank 18, and a suctioning pump 63. The culture fluid supply tank 18 is an intermediary supplying means for supplying the culture fluid 4 to the wells 22. The suctioning pump 63 is an intermediary removing means for removing the surplus volume in the culture fluid 4 that has been supplied to the wells 22.

The culture fluid supplying tank 18 filled with the culture fluid 4 supplies the culture fluid 4 to the culture fluid bath 8 through a silicon tube 64 and a hard pipe 65 set at the tip of the silicon tube 64.

A partition 8a of a particular height is provided to the culture fluid bath 8 of the holding petri dish 61, and if the culture fluid 4 continues to be supplied even after the level of the culture fluid 4 has reached the height of the partition 8a, the volume supplied after that moment is stored in a surplus culture fluid trapping unit 8b.

The suctioning pump 63 suctions the culture fluid 4 in the surplus culture fluid trapping unit 8b through a silicon tube 66 so as to cause it to flow to a waste liquid storing unit (not depicted). It is noted that the egg cell 3 tends to be below the level of the height of the partition 8a with the culture fluid 4 being suctioned through the channel 5, and thus only the culture fluid 4 can be suctioned through the silicon tube 66.

Additionally, the holding petri dish 61 in the present embodiment as well is provided with the connection unit 32, which is a pipe made of a hard material, so that the culture fluid 4 in the well 22 is suctioned/discharged via the connection unit 32 and the silicon tube 13 into which the connection unit 32 is press-fitted.

Also in the present embodiment explained above, the egg cell 3 is held via suction on the opening 5a formed on the side wall 22a of the well 22 provided on a transparent material. Accordingly, the present embodiment as well enables the picking up of a clear image of the egg cell 3 held via suction.

Generally, when genes are introduced to the egg cells 3, approximately ten through fifty egg cells are processed in a single test in order to increase the accuracy of the data for gene research. The introduction operation sometimes takes more than thirty minutes when genes are introduced while adjusting the orientation of the egg cells, and in such a case, the culture fluid 4 needs to be suctioned slightly and continuously in order to keep being held. This causes a risk that the amount of culture fluid 4 in the well 22 will decrease, resulting in a drying out so that the egg cell 3 is damaged.

However, as the microinjection apparatus 60 in the present embodiment includes a culture fluid supplying tank 18 for supplying the culture fluid 4 to the well 22 and the suctioning pump 63 to remove a surplus volume in the well 22, the culture fluid 4 is prevented from decreasing too much or from drying out to damage the egg cell 3 even when the slight suctioning of the culture fluid 4 from the well 22 continues during an injection operation lasting for a long time.

Also, in the present embodiment, the culture fluid bath 8 includes the surplus culture fluid trapping unit 8b, and the suctioning pump 63 suctions the culture fluid 4 in the surplus culture fluid trapping unit 8b. This makes it possible to remove a surplus volume in the culture fluid 4 effectively.

### <Seventh Embodiment>

In the explanation of the present embodiment, the same or similar parts (elements) as in the first through sixth embodiments are denoted by the same symbols as in those embodiments, and the explanations thereof will be omitted.

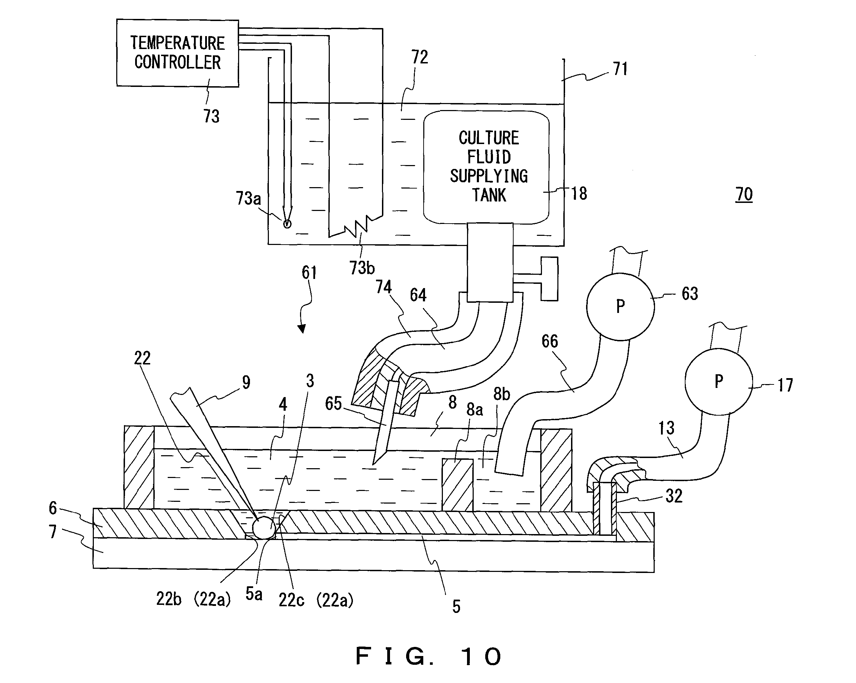

Fig. 10 is a cross-sectional view depicting the main part of a microinjection apparatus 70 according to the seventh embodiment.

The microinjection apparatus 70 according to the present embodiment includes the culture fluid supplying tank 18 in a temperature control bath (water bath) 71. The temperature control bath 71 is full of a liquid 72 used for controlling the temperature. A temperature controller 73 is provided, as temperature adjustment means for adjusting the temperature of the culture fluid 4, outside of the temperature control bath 71 in such a manner that a temperature measurement unit 73a and a heater 73b of the temperature controller 73 are in the liquid 72.

This configuration makes it possible to adjust the temperature of the culture fluid 4 to be supplied to the culture fluid supplying tank 18, and thereby to adjust the temperature of the culture fluid 4 in the culture fluid bath 8.

In addition, the silicon tube 64 connected to the culture fluid supplying tank 18 is coated with a heat insulation material 74 so that the temperature of the culture fluid 4 is not affected by ambient temperature.

Also in the present embodiment explained above, the egg cell 3 is held via suction on the opening 5a formed on the side wall 22a of the well 22 provided on a transparent material. Accordingly, the present embodiment as well enables the picking up of a clear image of the egg cell 3 held via suction.

Also, in the present embodiment the microinjection apparatus 70 includes the liquid 72 for controlling the temperature of the culture fluid 4 to be supplied to the culture fluid supplying tank 18. This makes it possible to adjust the temperature of the culture fluid 4, to a temperature suitable for the egg cells (introduced-substance receiving bodies) and for the introduced substances.

Also, in the present embodiment the silicon tube 64 connected to the culture fluid supplying tank 18 is coated with the heat insulation material 74. This configuration prevents the culture fluid 4 in the silicon tube 64 from being affected by ambient temperature, enabling the temperature to be adjusted more effectively.

### <Eighth Embodiment>

In the explanation of the present embodiment, only the parts of the entire configuration of the above described microinjection apparatus that have not been explained will be explained. Also, in the explanation of the present embodiment, the same or similar parts (elements) as in the first through seventh embodiments are denoted by the same symbols as in those embodiments, and the explanations thereof will be omitted.

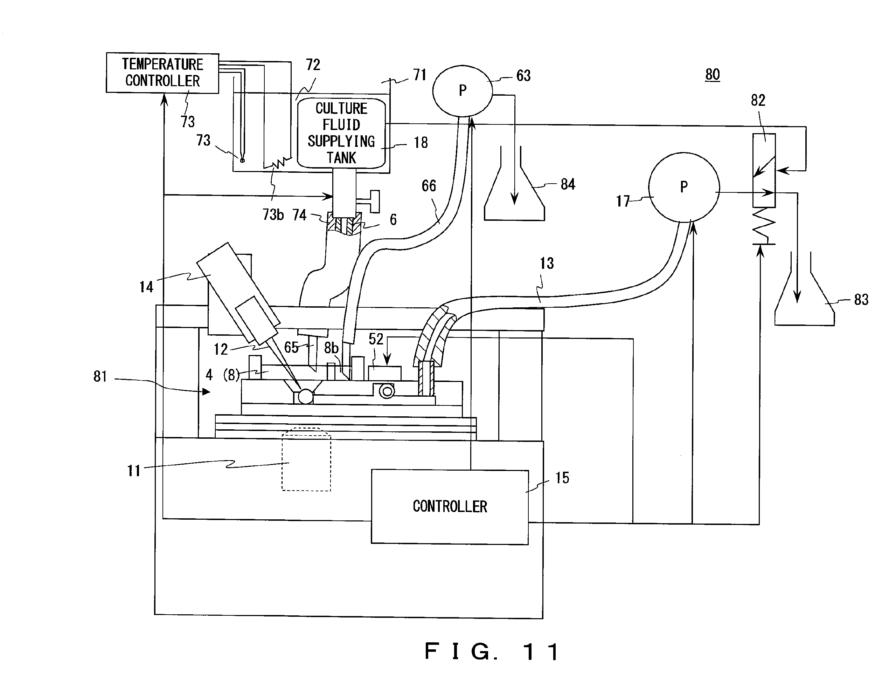

Fig. 11 depicts the entire configuration of a microinjection apparatus 80 according to the eighth embodiment of the present invention.

The controller 15 of the microinjection apparatus 80 is connected not only to the suction/discharge pump 17, the culture fluid supplying tank 18, the electromagnet 52, the suctioning pump 63, and the temperature controller 73, but also to an electromagnetic valve 82.

The electromagnetic valve 82 opens and closes the channel 5 for the culture fluid 4 being supplied by the suction/discharge pump 17 from the culture fluid supplying tank 18 and the channel 5 for the culture fluid 4 being suctioned by the suction/discharge pump 17. The culture fluid 4 suctioned by the suction/discharge pump 17 is stored in a waste liquid storing unit 83.

The culture fluid 4 suctioned by the suctioning pump 63 from the surplus culture fluid trapping unit 8b in the culture fluid bath 8 is stored in a waste liquid storing unit 84 that is provided separately from the waste liquid storing unit 83.

As described in the above explanations of the first through the eighth embodiments, the holding petri dish disclosed herein makes the introduced-substance receiving body held via suction at an opening formed on the side wall of each of the wells be formed on a transparent material. This configuration can prevent a situation in which the observation of an introduced-substance receiving body is hindered by channels or opaque materials when images of the introduced-substance receiving body held on the opening are to be picked up. Thereby, the disclosed holding petri dish enables the picking up of a clear image of an introduced-substance receiving body that is being held via suction.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a depicting of the superiority and inferiority of the invention. Although the embodiments of the present inventions have been described in detail, it should be understood that various changes, substitutions, and alterations could be made hereto without departing from the scope of the invention.

## Claims

1. A holding petri dish (1, 21) used for a process of microinjection for introducing an introduced substance into an introduced-substance receiving body (3), comprising:
a well (2, 22) provided on a transparent material (7); and
a channel (5) including an opening (5a) for holding the introduced-substance receiving body (3) via suction, said opening (5a) being disposed on a side wall (2a, 22a) of the well (2, 22).

2. The holding petri dish (1, 21) according to claim 1, wherein:
the side wall (22a) of the well (22) includes an inclined plane (22c) whose diameter gradually increases with increasing distance from a bottom side toward a top side, and a vertical plane (22b) being disposed below the inclined plane (22c) and including the opening (5a).

3. The holding petri dish (1, 21) according to claim 1 or 2, wherein:
a side wall (22a') of the well (22') is at least partially made of a multi-sided pyramid whose diameter gradually increases with increasing distance from a bottom side toward a top side or is made of a curved and inclined plane (22c').

4. The holding petri dish (1, 21) according to any preceding claim, wherein:
the side wall (22a") includes an inclined plane (22c''-1) whose diameter gradually increases with increasing distance from a bottom side toward a top side, and a plane that is disposed on a side of the opening (5a) and that is opposite to the inclined plane (22c''-1); and
the plane opposite to the inclined plane is a vertical plane (22c''-2) or a plane inclined at a smaller angle with respect to the vertical plane than the angle of incline of the inclined plane.

5. The holding petri dish (1, 21) according to claim 4, wherein:
the plane opposite to the inclined plane is the vertical plane; and
the inclined planes (22c''-1 and 22c'''-1) and the vertical planes (22c''-2 and 22c'''-2) are semi-elliptical cones or semi-multi-sided pyramids with corners R.

6. The holding petri dish (1, 21) according to any preceding claim, further comprising:
a connection unit (32) disposed between a suction-mechanism-side pipe (13) connected to a suction mechanism for holding the introduced-substance receiving body (3) via suction through the channel (5) and the channel (5).

7. The holding petri dish (1, 21) according to claim 6, wherein:
the connection unit (32) is a pipe made of a hard material.

8. A microinjection apparatus (10) for introducing an introduced substance into an introduced-substance receiving body (3), comprising:
a holding petri dish (1) including a well (2) provided on a transparent material (7), and a channel (5) having an opening (5a) for holding the introduced-substance receiving body (3), said opening (5a) being disposed on a side wall (2a) of the well (2);
a suction mechanism (16) for holding the introduced-substance receiving body (3) on the opening (2a) of the well (2) via suction through the channel (5); and
a transmission observation optical system (11) for performing transmission observation of the introduced-substance receiving body (3).

9. The microinjection apparatus (10) according to claim 8, further comprising:
a discharge mechanism (16) for discharging an intermediary (4) to the introduced-substance receiving body (3).

10. The microinjection apparatus (10) according to claim 9, wherein:
a width (D1) of a part having the opening (5a) in the well (2) of the holding petri dish (1) is 1.5 through 5 times greater than a size of the introduced-substance receiving body (3).

11. The microinjection apparatus (10) according to claim 9 or 10, wherein:
the discharge mechanism (16) discharges the intermediary (4) to the introduced-substance receiving body (3) through the channel (5) of the holding petri dish (1).

12. The microinjection apparatus (10) according to claim 11, wherein:
the holding petri dish (1) includes a plurality of the wells (2) and a plurality of the channels (5); and
the suction mechanism (16) and the discharge mechanism (16) are provided to each of the channels (5).

13. The microinjection apparatus (10) according to any of claims 8 to 12, wherein:
the holding petri dish (1) further includes opening/closing mechanisms (52, 53) being provided to the channel (5) for opening and closing the channel (5).

14. The microinjection apparatus (10) according to any of claims 8 to 13, wherein:
the transmission observation optical system (11) is disposed below the well (2).

15. A microinjection method of introducing an introduced substance into an introduced-substance receiving body (3), comprising:
a suctioning step of holding, via suction, the introduced-substance receiving body (3) on an opening (5a) provided on a side wall (2a) of a well (2), said introduced-substance receiving body (3) having been guided to the well (2) on a transparent material (7) in a holding petri dish (1); and
an observing step of performing transmission observation of the introduced-substance receiving body (3) held on the opening (5a) of the well (2).
